# EUROPÄISCHE PATENTANMELDUNG

(11) **EP 0 972 765 A1**
(43) Veröffentlichungstag der Anmeldung: **19.01.2000**
(21) Anmeldenummer: 99112438.9
(22) Anmeldetag: 30.06.1999
(51) Int. Cl.: C07D 213/06

(54) **Verfahren zur Herstellung von Arylpyridinen**

(30) Priorität: 11.07.1998 DE 19831246
(71) Anmelder: Clariant GmbH, 65929 Frankfurt am Main (DE)
(72) Erfinder: Nörenberg, Antje, Dr., 64572 Büttelborn (DE); Haber, Steffen, Dr., 61462 Königstein (DE); Meudt, Andreas, Dr., 65439 Flörsheim-Weilbach (DE)

(57) **Zusammenfassung**

2-, 3- oder 4-Arylpyridine werden hergestellt, indem ein Halogenpyridin mit einer Arylgrignardverbindung, wobei Halogen Chlor oder Brom bedeutet, in Gegenwart eines Palladiumkatalysators der Formel (IV) 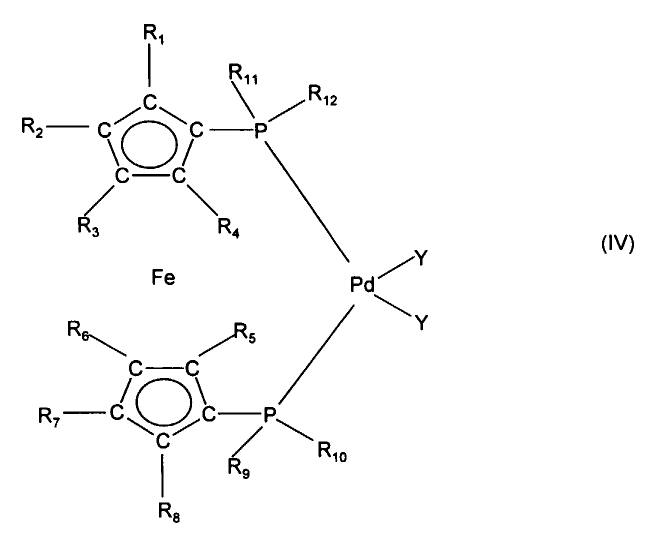 umgesetzt wird.

## Beschreibung

Die vorliegende Erfindung betrifft ein neues Verfahren zur Herstellung von Arylpyridinen aus Halogenpyridinen und Arylgrignardreagenzien mit einem Phosphino-palladium-ferrocen-Katalysator. Arylpyridine haben technische Bedeutung als Wirkstoffvorprodukte für den Agrobereich.

Eine häufig angewandte Methode zur Synthese von Arylpyridinen im Labormaßstab sind palladiumkatalysierte Kreuzkupplungen, bei der Iod- und Bromaromaten mit metallorganischen Arylderivaten, insbesondere Arylboronsäuren oder Arylgrignardreagenzien in Gegenwart von Palladium- oder Nickelkatalysatoren umgesetzt werden. Beispiele, die diese Methodik beschreiben, findet man beispielsweise in E. Negishi, F.-T. Luo, R. Frisbee, H. Matsushita, Heterocycles 18, 1982, 117; T. Sakamoto, Y. Kondo, N. Murata, H. Yamanaka, Tetrahedron 49, 1993, 9713, EP 0 834 508 A1 und WO 96/21647.

Trotz der Vielzahl von Veröffentlichungen auf dem Gebiet der Synthese von Arylpyridinen in Gegenwart von Nickel- bzw. Palladiumkatalysatoren sind bisher keine Beispiele für eine größere technische Umsetzung der Methoden bekannt. Dies ist darauf zurückzuführen, daß die beschriebenen Katalysatorsysteme häufig nur mit nicht ökonomischen Katalysatormengen oder geringen Selektivitäten, d.h. hohen Raten an Dimerisierungsprodukten, einsetzbar sind. Ansonsten müssen große Mengen an Katalysator - allgemein > 1 mol % - zugesetzt werden, um technisch nutzbare Umsätze zu erzielen. Aufgrund der Komplexität der Reaktionsgemische ist zudem kein einfaches Katalysatorrecycling möglich, so daß Katalysatorkosten in der Regel eine technische Realisierung verhindern.

Außerdem beobachtet man bei der Suzuki-Kupplung substituierter Biphenyle mit gängigen Katalysatorsystemen, wie Pd(Oac)₂/Triphenylphosphan-Gemischen, als Nebenreaktion Arylübertragungen (D:F: O'Keefe et al., Tetrahedron Letters 1992, 6679).

Aus den genannten Gründen ist es von großem industriellem Interesse, bessere, technisch nutzbare Katalysatorsysteme für die Synthese von Arylpyridinen, insbesondere für die Arylierung von ökonomisch günstigen Brom- und Chlorpyridinen zu finden. Es bestand somit ein großer Bedarf nach einem Verfahren, das die beschriebenen Nachteile vermeidet und Arylpyridine in hoher Reinheit in technisch einfacher Weise zugänglich macht.
Diese Aufgabe wird gelöst durch ein Verfahren zur Herstellung von Arylpyridinen der Formel (I) 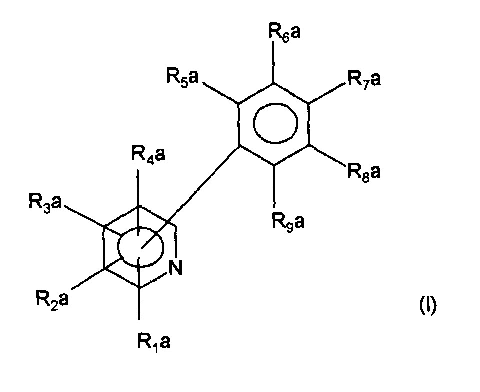 worin
R₁ₐ bis R₉ₐ gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, C₁-C₁₂-Acyloxy, O-Phenyl, O-Benzyl, Aryl, Heteroaryl, F, Cl, NO₂, CN, SO₂R, SOR, wobei R Aryl, vorzugsweise Phenyl oder Naphthyl, F oder CₙF₂ₙ₊₁ mit n = 1 bis 12, ist, NH(C₁-C₆-alkyl), N(C₁-C₆-alkyl)₂, CH=N(C₁-C₆-alkyl), CX₃, wobei X F, Cl oder Br ist, COO-(C₁-C₁₂-alkyl), CO-(C₁-C₁₂-alkyl), CO-Phenyl, COO-Phenyl, CON(C₁-C₈-alkyl)₂, CONH(C₁-C₈-alkyl), CHCHCOO-(C₁-C₁₂-alkyl), PO(Phenyl)₂, PO-(C₁-C₈-alkyl)₂ oder PO₃-(C₁-C₈-alkyl)₂ haben,
   dadurch gekennzeichnet, daß ein Halogenpyridin der Formel (II) 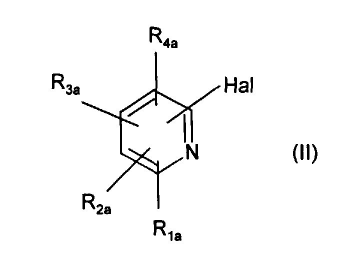 mit einer Arylgrignardverbindung der Formel (III) 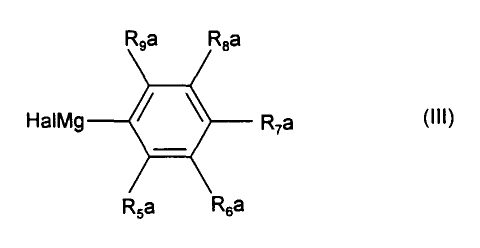 worin Hal für Chlor oder Brom steht, in Gegenwart eines Palladiumkatalysators der der Formel (IV) 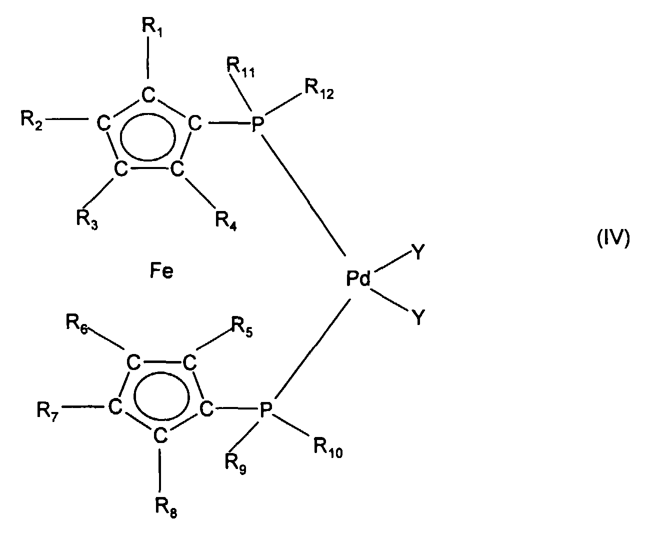 worin
   R₁ bis R₈ gleich oder verschieden sind und Wasserstoff, (C₁-C₄)-Alkyl, (C₅-C₈)-Cycloalkyl, (C₁-C₄-Alkoxy, Fluor, NH₂, NH-(C₁-C₄-Alkyl), N(C₁-C₄-alkyl)₂, CO₂-Alkyl-(C₁-C₄) oder Phenyl bedeuten, oder R₁ und R₂, oder R₂ und R₃, oder R₃ und R₄; und/oder R₅ und R₆, oder R₆ und R₇, oder R₇ und R₈ zusammen einen aliphatischen oder aromatischen Ring bilden,
   R₉ bis R₁₂ gleich oder verschieden sind und C₁-C₈-Alkyl, C₃-C₁₂-Cycloalkyl oder Aryl, das durch 1 bis 3 Substituenten aus der Gruppe C₁-C₄-Alkyl, C₁-C₄-Alkoxy und Halogen substituiert sein kann, bedeuten, und
   Y ein Anion einer organischen oder anorganischen Säure ist, umgesetzt wird.

Die Verbindung der Formel (I) kann ein 2-, 3- oder 4-Arylpyridin sein.

Bevorzugt ist ein Verfahren zur Herstellung von Verbindungen der Formel (I), worin R₁ₐ bis R₉ₐ Wasserstoff, C₁-C₈-Alkyl, C₂-C₈-Alkenyl, C₂-C₈-Alkinyl, C₁-C₈-Alkoxy, C₁-C₈-Acyloxy, F, Cl, CN, O-Phenyl, Phenyl, ein 5- oder 6-gliedriges Heteroaryl mit 1 bis 3 Heteroatomen aus der Gruppe O, S und N, COO-(C₁-C₈-alkyl), CO-(C₁-C₈-alkyl), CHCHCOO-(C₁-C₈-alkyl), CONH(C₁-C₄-alkyl) oder CON(C₁-C₄-alkyl)₂ bedeuten.

Besonders bevorzugt ist ein Verfahren zur Herstellung von Verbindungen der Formel (I), worin
R₁ₐ bis R₉ₐ Wasserstoff, C₁-C₄-Alkyl, C₁-C₄-Alkoxy, CN, COO(C₁-C₄-alkyl), Phenyl, Thiophenyl, Furanyl, Imidazolyl, Oxadiazolyl, Thiadiazolyl, Oxazolyl, 4,5-Dihydrooxazolyl, Pyridyl, CONH(C₁-C₂-alkyl), CON(C₁-C₂-alkyl)₂, F oder Cl bedeuten.

Von ganz besonderem Interesse ist ein Verfahren zur Herstellung von 2-Phenylpyridin.

Bevorzugt sind Katalysatoren der Formel (IV), worin R₁ bis R₈ Wasserstoff, Methyl, Ethyl, (C₅-C₆)-Cycloalkyl, Methoxy, Ethoxy, Fluor, NH(C₁-C₂-alkyl), N(C₁-C₂-alkyl)₂, Phenyl,
und R₉, R₁₀, R₁₁, R₁₂ Phenyl, Tolyl, Xylyl, Mesityl, Fluorphenyl oder (C₁-C₄)-Alkoxyphenyl bedeuten,
und Y für Chlorid, Bromid, Jodid, Fluorid, Acetat, Propionat, Benzoat, Sulfat, Hydrogensulfat, Nitrat, Phosphat, Tetrafluoroborat, Tosylat, Mesylat, Acetylacetonat, Hexafluoracetylacetonat oder Pyrazolyl steht.

Besonders bevorzugt sind Verbindungen der Formel (IV) worin R₁ bis R₈ für H, Methyl oder Phenyl, und R₉ bis R₁₂ für Phenyl, Tolyl, Xylyl, Fluorphenyl oder Methoxyphenyl stehen.

Ganz besonders bevorzugt sind
1,1'-Bis(diphenylphosphino)ferrocenpalladium(II)-chlorid (= Pd (dppf)Cl₂),
1,1'-Bis(diphenylphosphino)ferrocenpalladium(II)-chloride·Dichlormethan und
1,1'-Bis(diphenylphosphino)ferrocenpalladium(II)-bromid.

Der Katalysator wird, bezogen auf Halogenpyridin der Formel (II), zweckmäßig in der 0,00001 bis 1-fachen, vorzugsweise in der 0,0001 bis 0,1-fachen, insbesondere in der 0,0001 bis 0,08-fachen, molaren Menge eingesetzt.
Der Katalysator wird vorzugsweise in homogener Phase eingesetzt.
Als Lösungsmittel finden generell inerte organische Lösungsmittel Verwendung.
Bevorzugt werden aromatische, polar aprotische Lösungsmittel, wie Alkyl-, Dialkyl- oder Trialkylbenzole, oder Ether, wie Tetrahydrofuran, tert. Butylmethylether oder Diethylether, eingesetzt.

Die eingesetzten Palladiumkatalysatoren können vor der erfindungsgemäßen Umsetzung synthetisiert werden, sie können jedoch auch in situ ohne Verlust an katalytischer Aktivität erzeugt werden. Die Synthese des Katalysators erfolgt z.B. analog zu A. W. Rudie, D. W. Lichtenberg, M. L. Katcher, A. Davison, Inorg. Chem. 17, 1978, 2859.

Das erfindungsgemäße Verfahren wird im allgemeinen bei Temperaturen von 20 bis 200°C durchgeführt. Bevorzugt sind Temperaturen von 60 bis 180°C, insbesondere 60 bis 100°C.

Von besonderem Vorteil ist die Dosierung der Grignardkomponente, gelöst in einem inerten Lösungsmittel, zum Halogenaromaten und dem Katalysator in einem für alle Reaktionspartner inerten Lösungsmittel. Als inerte Lösungsmittel kommen vorzugsweise die vorstehend für den Katalysator genannten in Betracht.

Die eingesetzten Gridnardverbindungen werden vorteilhaft als 15 bis 40 gew.-%ige Lösungen in Tetrahydrofuran eingesetzt. Von besonderem Vorteil sind 20 bis 35 gew.-%ige Lösungen in Tetrahydrofuran.
Die molaren Mengenverhältnisse zwischen den Halogenpyridinen der Formel (II) und den Grignardverbindungen der Formel (III) betragen zweckmäßig 1:1 bis 1:1,3, vorzugsweise 1:1,001 bis 1:1,01.

Besonders 2-Phenylpyridin und dessen Derivate können mit Hilfe des erfindungsgemäßen Verfahrens in Ausbeuten größer als 95 % und mit Umsätzen und Selektivitäten > 98 % hergestellt werden. Nebenprodukte während der Reaktion, gebildet durch Dimerisierung der Grignardkomponente oder Dimerisierung der Halogenpyridine oder durch Folgereaktionen der 2-Phenylpyridine mit überschüssigem Grignardreagenz, werden nur in untergeordnetem Maßstab (< 1 %) beobachtet.

Die als Ausgangsstoffe benötigten Verbindungen sind bekannt und können nach an sich bekannten Methoden hergestellt werden.

### Beispiel 1

216,5 g (3,0 mol) Tetrahydrofuran (THF), 113,5 g (1,0 mol) 2-Chlorpyridin und 0,24 g Pd(dppf)Cl₂ (0,03 Mol-%) werden in einem Kolben vorgelegt und unter Rühren auf 50°C erhitzt. Man dosiert langsam 510 g Phenylmagnesiumchlorid-Lösung in THF (27 %ig) zu und kühlt von außen, damit die Temperatur von 50°C nicht überschritten wird. Anschließend rührt man 90 min bei dieser Temperatur nach. Man hydrolysiert langsam mit 250 g Wasser und läßt auf Raumtemperatur abkühlen. Nach der Phasentrennung wird die wäßrige Phase mit 250 ml tert.-Butylmethylether ausgerührt. Nach erneuter Phasentrennung werden die organischen Phasen vereinigt und das Reaktionsgemisch bei 150 mbar eingeengt. Anschließend destilliert man bei Normaldruck, bis eine Sumpftemperatur von 80°C erreicht ist. Der Destillationsrückstand wird bei 10 mbar destilliert. Man erhält 152.0 g 2-Phenylpyridin (98,1 %), Gehalt 98,7 % (laut GC).

### Beispiele 2 bis 4

Analog zu Beispiel 1 werden aus den entsprechenden Ausgangsverbindungen erfindungsgemäß folgende Verbindungen synthetisiert:

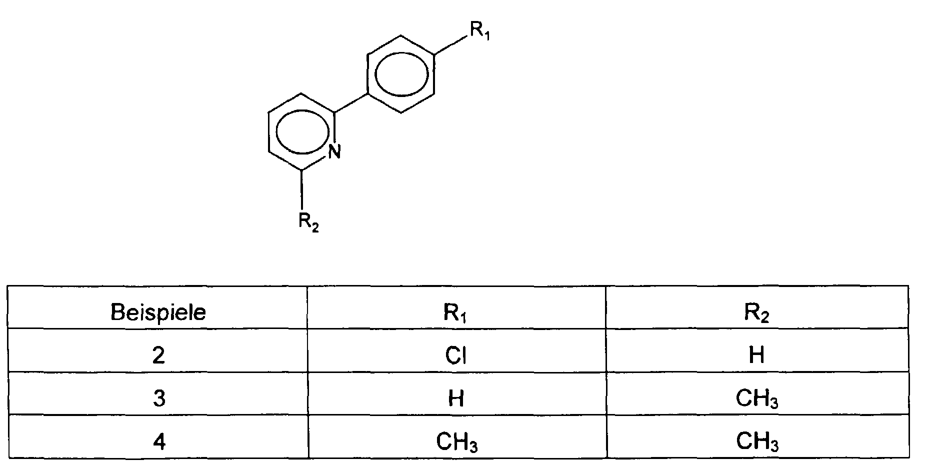

Die Ausbeuten betragen jeweils > 95 % und die Selektivität (laut GC) größer als 98%.

## Patentansprüche

1. Verfahren zur Herstellung von Arylpyridinen der Formel (I) 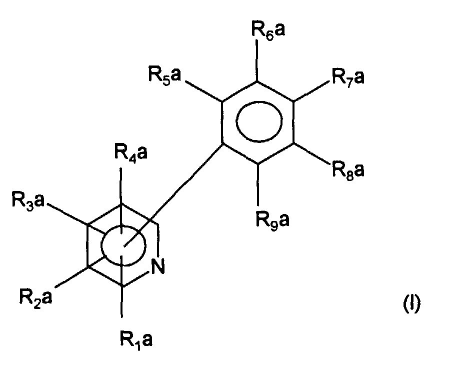 worin
R₁ₐ bis R₉ₐ gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, C₁-C₁₂-Acyloxy, O-Phenyl, O-Benzyl, Aryl, Heteroaryl, F, Cl, NO₂, CN, SO₂R, SOR, wobei R Aryl, F oder CₙF₂ₙ₊₁ mit n = 1 bis 12, ist, NH(C₁-C₆-alkyl), N(C₁-C₆-alkyl)₂, CH=N(C₁-C₆-alkyl), CX₃, wobei X F, Cl oder Br ist, COO-(C₁-C₁₂-alkyl), CO-(C₁-C₁₂-alkyl), CO-Phenyl, COO-Phenyl, CON(C₁-C₈-alkyl)₂, CONH(C₁-C₈-alkyl), CHCHCOO-(C₁-C₁₂-alkyl), PO(Phenyl)₂, PO-(C₁-C₈-alkyl)₂ oder PO₃-(C₁-C₈-alkyl)₂ haben,
dadurch gekennzeichnet, daß ein Halogenpyridin der Formel (II) 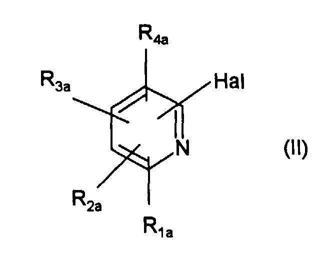 mit einer Arylgrignardverbindung der Formel (III) 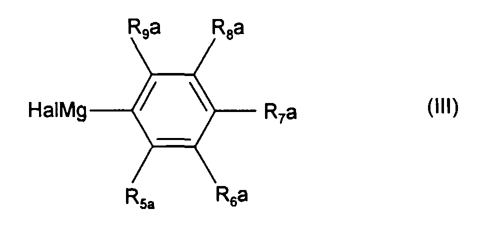 worin Hai für Chlor oder Brom steht, in Gegenwart eines Palladiumkatalysators der der Formel (IV) 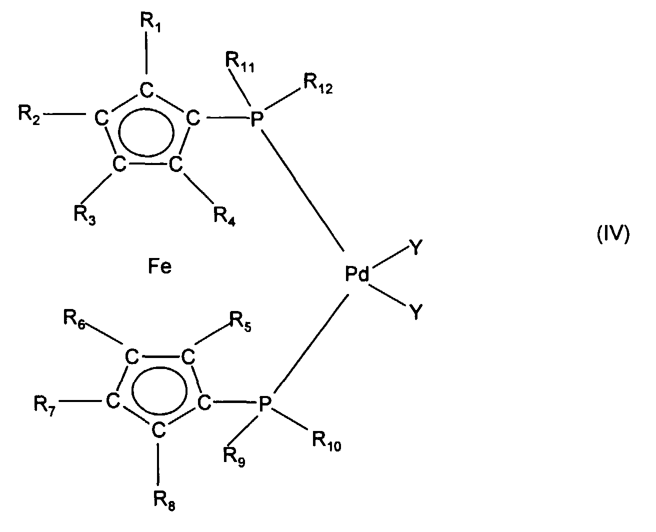 worin
R₁ bis R₈ gleich oder verschieden sind und Wasserstoff, (C₁-C₄-Alkyl, (C₅-C₈)-Cycloalkyl, (C₁-C₄)-Alkoxy, Fluor, NH₂, NH-(C₁-C₄-Alkyl), N(C₁-C₄-alkyl)₂, CO₂-Alkyl-(C₁-C₄) oder Phenyl bedeuten, oder R₁ und R₂, oder R₂ und R₃, oder R₃ und R₄; und/oder R₅ und R₆, oder R₆ und R₇, oder R₇ und R₈ zusammen einen aliphatischen oder aromatischen Ring bilden,
R₉ bis R₁₂ gleich oder verschieden sind und C₁-C₈-Alkyl, C₃-C₁₂-Cycloalkyl oder Aryl, das durch 1 bis 3 Substituenten aus der Gruppe C₁-C₄-Alkyl, C₁-C₄-Alkoxy und Halogen substituiert sein kann, bedeuten, und
Y ein Anion einer organischen oder anorganischen Säure ist, umgesetzt wird.

2. Verfahren nach Anspruch 1, dadurch gekennzeichnet, daß
R₁ₐ bis R₉ₐ Wasserstoff, C₁-C₈-Alkyl, C₂-C₈-Alkenyl, C₂-C₈-Alkinyl, C₁-C₈-Alkoxy, C₁-C₈-Acyloxy, F, Cl, CN, O-Phenyl, Phenyl, ein 5- oder 6-gliedriges Heteroaryl mit 1 bis 3 Heteroatomen aus der Gruppe O, S und N, COO-(C₁-C₈-alkyl), CO-(C₁-C₈-alkyl), CHCHCOO-(C₁-C₈-alkyl), CONH(C₁-C₄-alkyl) oder CON(C₁-C₄-alkyl)₂ bedeuten.

3. Verfahren nach Anspruch 1 oder 2, dadurch gekennzeichnet, daß
R₁ₐ bis R₉ₐ Wasserstoff, C₁-C₄-Alkyl, C₁-C₄-Alkoxy, CN, COO(C₁-C₄-alkyl), Phenyl, Thiophenyl, Furanyl, Imidazolyl, Oxadiazolyl, Thiadiazolyl, Oxazolyl, 4,5-Dihydrooxazolyl, Pyridyl, CONH(C₁-C₂-alkyl), CON(C₁-C₂-alkyl)₂, F oder Cl bedeuten.

4. Verfahren nach mindestens einem der Ansprüche 1 bis 3, dadurch gekennzeichnet, daß die Verbindung der Formel (I) 2-Phenylpyridin ist.

5. Verfahren nach mindestens einem der Ansprüche 1 bis 4, dadurch gekennzeichnet, daß in der Verbindung der Formel (IV)
R₁ bis R₈ Wasserstoff, Methyl, Ethyl, (C₅-C₆)-Cycloalkyl, Methoxy, Ethoxy, Fluor, NH(C₁-C₂-alkyl), N(C₁-C₂-alkyl)₂, Phenyl,
und R₉, R₁₀, R₁₁, R₁₂ Phenyl, Tolyl, Xylyl, Mesityl, Fluorphenyl oder (C₁-C₄)-Alkoxyphenyl bedeuten,
und Y für Chlorid, Bromid, Jodid, Fluorid, Acetat, Propionat, Benzoat, Sulfat, Hydrogensulfat, Nitrat, Phosphat, Tetrafluoroborat, Tosylat, Mesylat, Acetylacetonat, Hexafluoracetylacetonat oder Pyrazolyl steht.

6. Verfahren nach mindestens einem der Ansprüche 1 bis 5, dadurch gekennzeichnet, daß in der Verbindung der Formel (IV)
R₁ bis R₈ für H, CH₃ oder Phenyl, und R₉ bis R₁₂ für Phenyl, Tolyl, Xylyl, Fluorphenyl oder Methoxyphenyl stehen.

7. Verfahren nach mindestens einem der Ansprüche 1 bis 6, dadurch gekennzeichnet, daß der Katalysator der Formel (IV)
1,1'-Bis(diphenylphosphino)ferrocenpalladium(II)-chlorid,
1,1'-Bis(diphenylphosphino)ferrocenpalladium(II)-chlorid·Dichlormethan oder
1,1'-Bis(diphenylphosphino)ferrocenpalladium(II)-bromid ist.

8. Verfahren nach mindestens einem der Ansprüche 1 bis 7, dadurch gekennzeichnet, daß der Katalysator der Formel (IV) in einer 0,00001 bis 1-fachen, vorzugsweise 0,0001 bis 0,1-fachen, molaren Menge, bezogen auf das Halogenpyridin der Formel (II), eingesetzt wird.

9. Verfahren nach mindestens einem der Ansprüche 1 bis 8, dadurch gekennzeichnet, daß die Umsetzung in einem aromatischen, polar aprotischen Lösungsmittel oder einem Ether durchgeführt wird.

10. Verfahren nach einem oder mehreren der Ansprüche 1 bis 9, dadurch gekennzeichnet, daß die Arylgrignardverbindung der Formel (III) in einem inerten Lösungsmittel gelöst und zu dem Halogenpyridin der Formel (II) und dem Katalysator der Formel (IV) zudosiert wird.
